# EUROPEAN PATENT SPECIFICATION

(11) **EP 0 573 253 B1**
(45) Date of publication and mention of the grant of the patent: **22.08.2001**
(21) Application number: 93304238.4
(22) Date of filing: 01.06.1993
(51) Int. Cl.: A61K 7/06, A61K 7/48, A61K 31/22, A61K 31/23, A61K 31/215

(54) **Topical anti-bacterial composition**
Topisches antibakterielles Mittel
Composition topique antibactérienne

(30) Priority: 03.06.1992 GB 9211725; 25.02.1993 GB 9303816
(43) Date of publication of application: 08.12.1993
(73) Proprietor: UNILEVER PLC, London EC4P 4BQ (GB); UNILEVER N.V., 3013 AL Rotterdam (NL)
(72) Inventor: Gibson, Walter T., c/o Unilever Res. Colworth Lab., Sharnbrook, Bedford MK44 1LQ (GB); Hagan, Desmond B., Unilever Res. Port Sunlight Lab, Wirral, Merseyside 63 3JW (GB); Suita-Mangano, Patricia, Unilever Research US Inc, Edgewater, New Jersey 07020 (US); Westgate, Gillian E., Unilever Res. Colworth Lab., Sharnbrook, Bedford MK44 1LQ (GB)
(74) Representative: Mulder, Cornelis Willem Reinier, Dr.

(56) References cited:
- EP-A- 0 155 333
- EP-A- 0 297 310
- EP-A- 0 437 006
- EP-A- 0 572 271
- WO-A-88/06880
- WO-A-92/10995
- DE-U- 9 206 336
- FR-A- 2 342 725
- US-A- 3 250 681
- US-A- 3 275 503
- US-A- 3 728 447
- US-A- 5 017 366
- CHEMICAL ABSTRACTS, vol. 89, no. 14, 2 October 1978, Columbus, Ohio, US; abstract no. 117515, LAWRENCE ET AL 'acyl lactylates in cosmetics' & DRUG COSMET. IND. vol. 122, no. 5 , 1978 , GB
- COSMETICS & TOILETRIES vol. 95 , April 1980 pages 43 - 45 LAWRENCE ET AL 'use of fatty acid lactylates in emulsification'

## Description

### Field of the invention

This invention relates to cosmetic compositions, methods of making such compositions, a method of preventing and/or reducing hair loss, and a method of treating skin conditions.

### Background of the Invention

Despite a considerable research effort, androgenetic alopecia (AGA) remains a common skin condition.

Previous work has demonstrated that photoactivated human protoporphyrins may activate complement (Lim & Gigli, 1981). Further recent findings (Young et al., 1990, Divaris et al, 1990) have suggested that AGA may be caused, at least in part, by an inflammatory process initiated by the activation of complement by ultraviolet A (UVA)-activated protoporphyrins. It is known that certain bacteria, which can live on human skin, are able to produce porphyrins and it has been postulated that these porphyrins of microbial origin are responsible for activating complement and triggering an inflammatory response which leads to hair loss (Trevino et al., 1991 Clinical Research 39, SID Abstracts, A537 and Vittimberga & Weinstock, Clinical Research 39, SID Abstracts A676).

In particular, the organism Propionibacterium acnes, which can colonise the human pilosebaceous duct and is known to produce porphyrins, is considered a possible contributory factor to the causation of AGA.

Despite these advances in the understanding of possible mechanisms operating in the complex pathology of AGA, a cure or preventative measure is not yet available.

Other cosmetic skin conditions, such as spots, pimples and boils, and in particular, acnes comedones, await an effective method of treatment.

Acyl lactylates are a known group of compounds. Some of them have been shown to possess preservative activity in foods and on leather and to be active against skin fungi (US 3 275 503). In particular, capryl-2 lactylate is said to be effective in preventing growth of the fungus Neurospora sitophila (CA 782605), and the unsaturated compound undecenoyl lactylate is stated to possess comedolytic activity (EP 0 443 956), although it is not suggested that the compound has any anti-microbial activity. FR 2342725, US 3275503 and EP 572271 describes the use of acyl lactylates as antimicrobial agents. US 3728447 describes the use of acyl lactylates for conditioning hair.

### Summary of the Invention

In a first aspect the invention provides a cosmetic method of preventing and/or treating hair loss comprising the application to the skin and/or hair of an effective amount of a cosmetic composition comprising a C₆-C₁₂ acyl lactylate or a derivative thereof having the structure

(RCO-(O-CHCH₃-CO)ₐ O)_{b} M

where:
RCO represents a (saturated) C₆ to C₁₂ acyl radical group;
M represents H or a cosmetically acceptable counterion having a valency of 1 or 2;
a is an integer from 1 to 4 (inclusive); and
b is 1 or 2

Whilst it has been shown previously (US 3 275 503), that calcium capryl-2 lactylate possesses growth-inhibiting properties for fungi (which, of course, are eukaryotic) the present inventors have found that the presently defined C₆-C₁₂ acyl lactylates provide a marked and beneficial inhibitory effect with regard to the growth of bacteria, and the growth of Propionibacterium acnes in particular. The present invention has been found to be especially beneficial in treatment of skin conditions, particularly when used on the scalp of men, women or children experiencing unwanted hair loss. Trials by the present applicants have shown that the presence of naturally occurring P. acnes on the human scalp can have the effect of accelerating hair loss.

A number of derivatives of these compounds can be envisaged (for example, compounds containing one or more substitutions in the acyl radical group) and these are intended to fall within the scope of the present invention.

Preferably the acyl lactylate compounds of the composition are those where M is H or a counterion elected from the group consisting of sodium, potassium or calcium.

It is also preferable that the acyl radical group contains 8 or 12 carbon atoms. Examples of such compounds are octanoyl and dodecanoyl lactylic acids. The most preferred compounds are the sodium, potassium or calcium salts of octanoyl or dodecanoyl lactylic acid.

It is highly preferred that compositions in accordance with the invention further comprise an anti-inflammatory agent.

Compositions in accordance with the invention preferably possess one or more of the following characteristics: the composition comprises a hair growth promoter; the composition comprises a substance acting as a sunscreen which is particularly effective at screening against ultraviolet A (UVA) radiation; and the composition comprises an anti-bacterial substance which is particularly active against P. acnes.

In another aspect the invention provides a method of treating a skin condition, comprising the application to the skin of a person of an effective amount of the composition of the invention.

Typically the skin condition will involve a local bacterial infection of the skin, pores or hair follicles, resulting in, for example, spots, pimples, boils, or, particularly, acne. Generally such conditions will be caused or exacerbated by, or be associated with, P. acnes.

In a further aspect the invention provides a method of preventing and/or treating hair loss, particularly hair loss due to androgenetic alopetia, comprising the application to the skin and/or hair of a person of an effective amount of the composition of the invention.

In still a further aspect the invention provides a method of making a composition for the treatment of skin conditions, comprising mixing a C₆-C₁₂ acyl lactylate as defined above with a cosmetically acceptable carrier to make a formulation suitable for topical application to the skin.

In another aspect the invention comprises the use of a C₆C₁₂ acyl lactylate as defined above in a cosmetic composition for the purpose of treating a skin condition.

A composition in accordance with the invention preferably comprises a C₆-C₁₂ acyl lactylate in the range 0.01-10% by weight, more preferably in the range 0.1-3%. The composition may advantageously contain one or more additional anti-bacterial substances.

A range of anti-bacterial substances are suitable for inclusion in the compositions of the invention. In addition to acyl lactylates and azelaic acid, other presently preferred compounds include:-

Chlorhexidine, Triclosan, Octopirox, zinc PTO, Irgasan DP 300, benzalkonium chloride, benzethonium chloride, phenol and other mild bactericides, estradiol, other follicular hormones, diphenhydramine HCl, chlorpheniramine maleate and other antihistamines; light sensitive compounds, chlorophyllin derivatives, cholesterol, salicylic acid, cystine, red pepper tincture, benzyl nicotinate, dl-menthol, 1-menthol, peppermint oil, calcium pantothenate, panthenol, castor oil, Hinokitiol, prednisolone, resorcinol, ethanol and other appropriate solvents.

Other suitable antimicrobial compounds include:
zinc oxide, benzoic acid, sulphur, proteolytic enzymes, vitamin A, L-asorbic acid monostearate, potassium asparaginate, almond oil, aluminium hydroxychloride, ammonia water, isopropyl myristate, allantoin, oleyl alcohol, citric acid, glycerin monostearate, ammonium glycyrrhizinate, whale tallow, beeswax, cysteine diethyl ester, sodium disulfonate salt of diphenylimidazone, dibutylhydroxytoluene, potassium hyroxide, sodium hydroxide, Sukuwaran, stearic acid, cetanol, L-serine, tocopherol, nicotinamide, butyl paraoxybenzoate, isopropylmethylphenol (biozol), biotin, chlorhexidine glyconate, polyoxyethylene styryl ether, polyethlene glycol, polysorbate 80, ioclecithin, laurylethylimidazoline disodium acetate, lauryldiethanolamide, pure lanolin, and linoleic acid.

Particularly preferred are Triclosan and Irgasan DP 300.

The appropriate concentration for use of each of these compounds varies according to their efficacy as bactericides and bacteristats, as well understood by those skilled in the art.

Similarly, there are numerous substances, both organic and inorganic, active as sunscreens suitable for topical application. Currently preferred compounds include the following:

| | Suggested Concentration (%) by weight |
|---|---|
| Pongamol | 1-10 |
| Oxybenzone | 2-6 |
| Sulisobenzone | 5-10 |
| Dioxybenzone | 3 |
| Methyl anthranilate | 3.5-5 |
| Butyl methoxydibenzoylmethane | 0.1-2.0 |
| Amino benzoic acid | 5-15 |
| Amyl dimethyl PABA | 1-5 |
| 2-Ethoxyethyl p-methoxy cinnamate | 1-3 |
| Diethanolamine p-methoxy cinnamate | 8-10 |
| Digalloyl trioleate | 2-5 |
| Ethyl 4-bis (hydroxypropyl) aminobenzoate | 1-5 |
| 2-Ethylexyl-2-cyano-3,3-diphenylacrylate | 7-10 |
| Ethylexyl p-methoxy cinnamate | 2-7.5 |
| 2-Ethylexyl salicylate | 3-5 |
| Glyceryl aminobenzoate | 2-3 |
| Homomenthyl salicylate | 4-15 |
| Lawsone with dihydroxyacetone | 0.25 with 33 |
| Octyl dimethyl PABA | 1.4-8 |
| 2-Phenylbenzimidazole-5-sulfonic acid | 1-4 |
| Triethanolamine salicylate | 5-12 |
| Red petrolatum | 30-100 |
| Titanium dioxide | 2-25 |

Preferably the substance active as a sunscreen is butyl methoxydibenzoylmethane. Other compounds suitable for use as sunscreen substances are shown in Table 1.

As mentioned previously, a composition in accordance with the invention preferably comprises a hair growth promoter. Examples include minoxidil, glucaraolactone, methionine, and glutamate and nicotinate esters. Appropriate concentrations for such hair growth promoters are in the range 0.2-2.0% by weight.

It is preferred that a composition in accordance with the invention comprises an anti-inflammatory agent. Such an anti-inflammatory agent may be steroidal or non-steroidal.

Examples of suitable steroidal agents include:
corticosteroids such as hydrocortisone hydroxyltriamcinolone, alpha-methyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone privalate, fluosinolone acetonide, fluocinonide, flucortine butylestene fluocortolone, fluprednidene (fluprednylidene) acetate, fluradrenolone, halcinonide, hydrocortisone acetate, hydrocortisone butyrate, methylprednisolene, triamcinolone acetonide, cortisone, cortodoxone, flucetonide fludrocortisone, difluorosone diacetate, fluradrenolone acetonide, medrysone, amcinafel, amcinafide, betamethasone and the balance of its esters, chloroprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof may be used. The preferred steroidal anti-inflammatory for use in the present invention is hydrocortisone.

Examples of suitable non-steroidal agents include the following:
the oxicams, such as piroxicam, isoxicam, tenoxicam, sudoxicam, the salicylates, such as aspirin, disalcid, benorylate, trilisate, safapryn, solprin, diflunisal, and fendosal; the acetic acid derivatives, such as diclofenac, fenlofenac, indomethacin, sulindac, tolmetin, isoxepac, furofenac, tiopinac, zidometacin, acemetacin, fentiazac, zomepirac, clidanac, oxepinac, and felbinac; the fenamates, such as mefenamic, meclofenamic, flufenamic, niflumic, and tolfenamic acids; the propionic acid derivatives, such as ibuprofen, naproxen, benoxaprofen, flurbiprofen, ketoprofen, fenoprofen, fenbufen, indoprofen, pirprofen, carprofen, oxaprozin, pranoprofen, miroprofen, tioxaprofen, suprofen, alminoprofen, and tiaprofenic; and the pyrazoles, such as phenylbutazone, oxyphenbutazone, feprazone, azapropazone.

Particularly preferred is ibuprofen.

The concentration of anti-inflammatory agent included in the composition naturally depends on the efficacy of the compound in question. However, the concentration of such agents will preferably be from 0.1% to 10% by weight, more preferably 0.5% to 5%.

The compositions of the invention preferably comprise a cosmetically acceptable carrier at 10-99.9% by weight, more preferably in the range 50-99%.

The compositions generally comprise up to 80% by weight of water, preferably 5-80%. Other optional ingredients include: an emulsifier (normally at 1-50% by weight, preferably 2-20% and more preferably 2-10%); surfactants (generally at 2-40% by weight, preferably from 5 to 30%); humectants; thickeners; colourants; perfumes; and preservatives.

Examples of chemical preservatives that may be included are:
formaldehyde; 2-bromo-2-nitropropane-1,3-diol; (e.g. Bronopol or Myacide BT); methyl paraben (e.g. Nipagin M); propyl paraben (e.g. Nipasol M); butyl paraben (e.g. Nipabutyl); imidazolidinyl urea (e.g. Germall 115); diazolidinyl urea (e.g. Germall II); sorbic acid/potassium sorbate; benzoic acid/sodium benzoate; salicylic acid/sodium salicylate; phenoxyethanol; phenoxyethanol (80%) + dibromodiacyanobutane (20%) (e.g. Euxyl K400); dimethylol dimethyl hydantoin (e.g. Glydant); Chloroacetamine; dehydroacetic acid; glutaraldehyde; methyl chloro isothiazolinone; phenyl ethyl acetate; 1-(3-chloroallyl)-3,5,7-triaza-1-azonia adamantane chloride (e.g. Quarternium 15); sodium hydroxymethylglycinate; and benzyl alcohol.

These preservatives can be employed individually or in mixtures.

The compositions of the invention may be formulated as liquids (e.g. as a lotion, conditioner, cream or gel) for use, if appropriate, in conjunction with an applicator such as a squeeze dropper, or a spray device (such as an aerosol can containing propellant or a contaner fitted with a pump to dispense the liquid). Alternatively, the compositions may be packaged in a container such as a tube, bottle or lidded jar, or provided as a liquid-impregnated fabric, such as a tissue wipe.

The compositions may also be in non-liquid form (e.g. powder, stick, paste or mousse).

### Experimental evidence of inhibition of P. acnes by C₆-C₁₂ acyl lactylates

Standard agar dilution tests in order to establish the minimum inhibitory concentration (% v/v) for C₆ acyl lactylate, C₈ acyl lactylate and C₁₂ acyl lactylate were performed both in the presence and the absence of a lipid on the following micro-organisms:
1. Propionibacterium acnes
2. Staphylococcus aureus
3. Escherichia coli
4. Candida albicans

Control experiments were performed employing benzoyl peroxide and erthyromycin.

The present tests were performed using Trypticase Soy Agar II incorporation plates with and without 10% intralipid, supplied by Kabi Pharmacia Inc. In each case the lactylate being tested was used at a starting concentration of 10,000 ug/ml.

The results in terms of minimum inhibitory concentration are given in Table II below.

As can be seen from the Table C₈ acyl lactylate is inhibitory against gram-negative bacteria at 1% v/v and inhibitory to gram-positive bacteria at 0.5 to 1% v/v. The minimum inhibitory concentration of C₈ acyl lactylate for P. acnes was 0.25% v/v in aqueous solution and 0.5% v/v in the presence of a lipid.

C₁₂ acyl lactylate showed no inhibitory effect at 1% v/v against gram-negative bacteria or against yeast. It was however extremely effective against gram-positive bacteria at 0.0625% v/v and outstandingly inhibitory against P. acnes having a minimum inhibitory concentration of 0.00039% v/v. Even in the presence of a lipid C₁₂ acyl lactylate was still shown to be remarkably effective at 0.25% v/v.

Both C₈ and C₁₂ acyl lactylate had similar pH effects, such that in the plates used the pH recorded was 5.5. At concentrations of 0.5% v/v and below, all pH readings were 7.0. A pH range of 5 to 8 allows for good bacterial growth of the strains tested. The Trypitcase Soy Agar plates employed were an unbuffered medium and thus the minimum inhibitory concentrations reading for C₈ and C₁₂ acyl lactylates were unaffected by pH at the 5 to 8 range.

In the case of C₆ acyl lactylate at a 15 v/v the pH recorded was 3.0 which had a direct inhibitory effect on the strains tested. At a concentration of 0.5% v/v however C₆ acyl lactylate exhibited anti-microbial inhibition against P. acnes in the presence of a lipid.

The present results thus show the effectiveness of especially C₈ and C₁₂ acyl lactylates against P. acnes. Of particular interest is their lipid compatability indicating that their inhibitory effect against P. acnes should be present in the water/oil emulsion conditions found in the pilo-sebaceous canal.

### Evaluation of Efficacy of Acyl Lactylates in Reducing the Rate of Hair Loss

The effect of selected acyl lactylates, as herein defined, on reducing the rate of hair loss was assessed using a panel of human volunteers.

Each panellist was issued with the same type of hair brush and received a hairbrushing demonstration. Each panellist was requested to brush his or her hair immediately on getting out of bed in the morning before other grooming took place. The hair brushing consisted of ten vigorous strokes over the entire head from front to back, ten strokes from back to front and then ten strokes from each side to the opposite side. Brushing was carried out over a towel or other light cloth to collect any hairs that fell. In addition, all the hairs on the brush were removed with forceps. The total number of hair collected each day was counted. Panellists were requested to continue with their routine hair shampooing and grooming techniques as usual, the only change to this routine being the application of products in accordance with the invention or corresponding control or placebo products.

During the first month, each panellist carried out only the standard brushing, collecting and counting of hairs lost to provide a basis for comparison later. For the second and third months, the scalp was treated by adding the test or placebo compositions in addition to the standard brushing technique as previously described.

The test and placebo compositions were provided in dropper bottles having a tubular applicator tip with a small orifice. Drops of liquid were applied to the scalp and gently rubbed in until the entire scalp was covered. On average, about 3 ml of liquid composition were applied per application. The test and placebo compositions were applied in this manner in the evening immediately before retiring for the night.

In this test, volunteer human panellists were divided into 2 groups, test and placebo formulations based on those of Examples 1 and 2 being applied as follows:
- Test Group A:: C₁₂ acyl lactylate insulin (i.e. as for Example 1)
- Placebo Group B:: Example 1 (or 2) formulation without growth factors.

Formulations as described were applied daily (at night) over a period of one month.

The results were analysed by the standard Student t-test. It was concluded that test composition A containing C₁₂ acyl lactylate showed a greater reduction in the rate of loss of hair than did the placebo composition B containing no acyl lactylate.

It was concluded from this experiment that C₁₂ acyl lactylate provided significant protection to the scalp against hair loss, such that the normal rate of hair loss was significantly reduced.

### Examples

The various aspects of the invention are illustrated by the following examples.

### Example 1

The lotion had the following formulation:

| | % w/w |
|---|---|
| octanoyl lactylic acid, sodium salt | 1 |
| ethanol | 20 |
| xanthan gum | 0.3 |
| water | to 100 |

### Example 2

This example illustrates a hair tonic which is particularly suitable for treating dandruff as well as reducing the rate of hair loss.

The hair tonic had the following formulation:

| | % w/w |
|---|---|
| dodecanoyl lactylic acid, potassium salt | 1 |
| ethanol | 20 |
| xanthan gum | 0.3 |
| water | to 100 |

### Example 3

This example illustrates a hair tonic which is suitable for enhancing the rate of hair growth as well as reducing the rate of hair loss.

The lotion had the following formulation:

| | % w/w |
|---|---|
| octanoyl lactylic acid, sodium salt | 1 |
| dodecanoyl lactylic acid, sodium salt | 1 |
| ethanol | 10 |
| xanthan gum | 0.3 |
| glucarolactone | 1 |
| ethane diol | 10 |
| water | to 100 |

**Table 1**

| CTFA Name | Trade Name | Supplier |
|---|---|---|
| Benzophenone-3 | UVINUL M-40 | BASF Chemical Co. |
| Benzophenone-4 | UVINUL MS-40 | BASF Chemical Co. |
| Benzophenone-8 | SPECRA-SORB UV-24 | American |
| | | Cyanamide |
| DEA | | |
| Methoxycinnamate | BERNEL HYDRO | Bernel Chemical |
| Ethyl dihydroxypropyl-PABA | AMERSCREEN P | Amerchol Corp. |
| Glyceryl PABA | NIPA G.M.P.A. | Nipa Labs. |
| Homosalate | KEMESTER HMS | Hunko Chemical |
| Methyl anthranilate | SUNAROME UVA | Felton Worldwide |
| Octocrylene | UVINUL N-539 | BASF Chemical Co. |
| Octyl dimethyl PABA | AMERSCOL | Amerschol Corp. |
| Octyl methoxycinnamate | PARSOL MCX | Givaudan |
| Octyl salicylate | SUNAROME WMO | Felton Worldwide |
| PABA | PABA | National Starch |
| 2-Phenylbenzimidazole-5-sulphonic acid | EUSOLEX 232 | EM Industries |
| TEA salicylate | SUNAROME W | Felton Worldwide |
| 3-(4-methylbenzylidene)-camphor | EUSOLEX 6300 | EM Industries |
| Benzophenone-1 | UVINUL 400 | BASF Chemical Co. |
| Benzophenone-2 | UVINUL D-50 | BASF Chemical Co. |
| Benzophenone-6 | UVINUL D-49 | BASF Chemical Co. |
| Benzophenone-12 | UVINUL 408 | BASF Chemical Co. |
| 4-Isopropyl dibenzoyl methane | EUSOLEX 8020 | EM Industries |
| Etocrylene | UVINUL N-35 | BASF Chemical Co. |

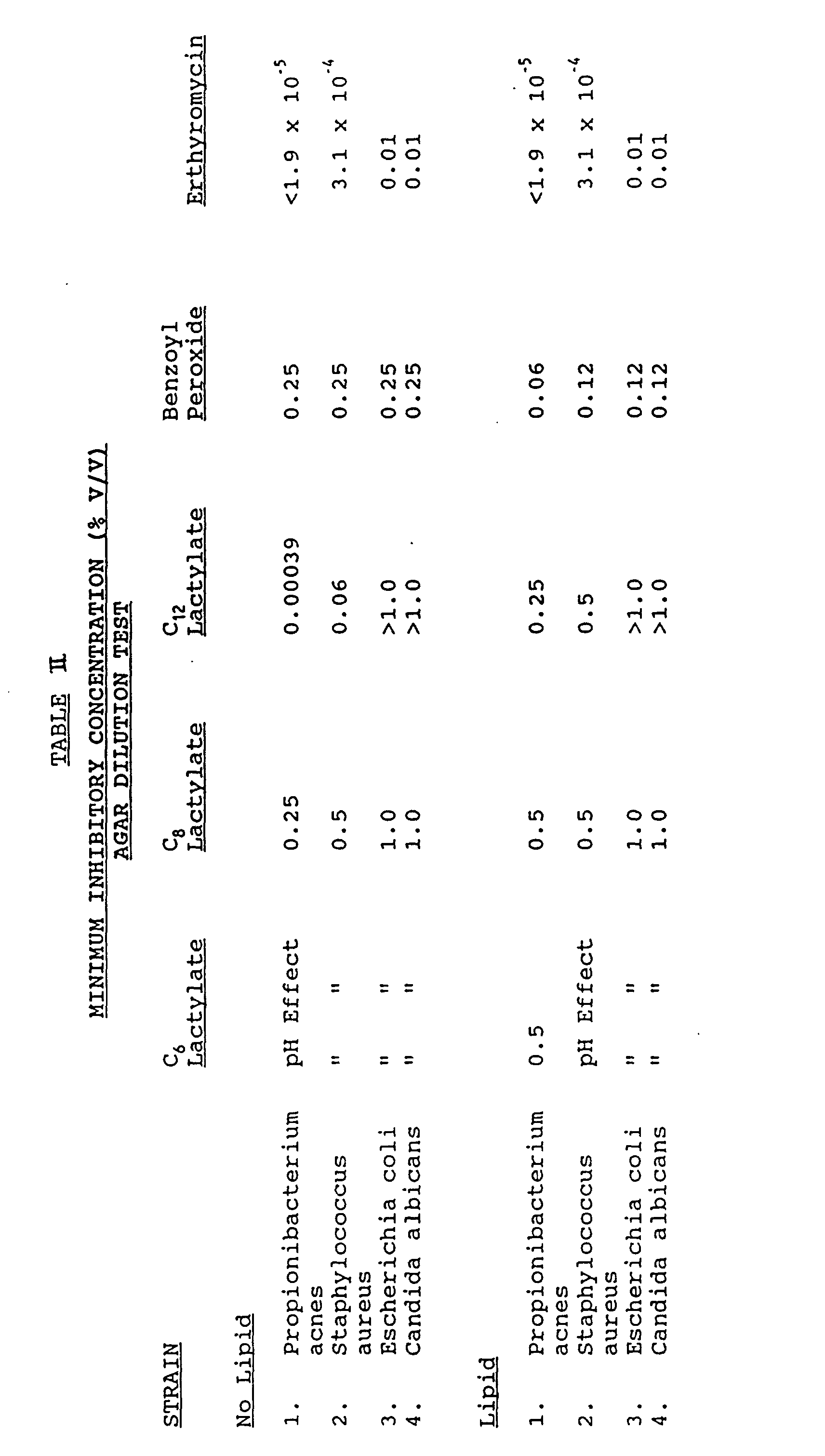

## Claims

1. A cosmetic method of preventing and/or treating hair loss comprising the application to the skin and/or hair of an effective amount of a cosmetic composition comprising a C₆-C₁₂ acyl lactylate or a derivative thereof having the structure
(RCO-(O-CHCH₃-CO)ₐO)_{b} M
where: RCO represents a saturated acyl radical group;
M represents H or a cosmetically acceptable counterion having a valency of 1 or 2;
a is an integer of 1 to 4; and
b is 1 or 2.

2. A cosmetic method according to claim 1 wherein the acyl lactylate is such that M is H or a counterion selected from the group consisting of sodium, potassium or calcium.

3. A cosmetic method according to claim 1 or 2 wherein the acyl lactylate is such that the acyl radical group contains 8 or 12 carbon atoms.

4. A cosmetic method according to claims 1, 2 or 3 wherein the composition further comprises a substance acting as a sunscreen.

5. A cosmetic method according to any one of claims 1-4 wherein the composition further comprises a hair growth promoter.

6. A cosmetic method according to any one of the preceding claims wherein the composition further comprises an anti-inflammatory agent.

7. A cosmetic method according to any one of the preceding claims, wherein the hair loss is caused or exacerbated by, or associated with, Propionibacterium acnes.

## Patentansprüche

1. Kosmetisches Verfahren zur Verhinderung und/oder Behandlung von Haarausfall, umfassend die Anwendung einer wirksamen Menge einer kosmetischen Zusammensetzung, die ein C₆-C₁₂-Acyllactylat oder ein Derivat davon mit der Struktur
(RCO-(O-CHCH₃-CO)ₐO)_{b} M ,
worin: RCO einen gesättigten Acylrest wiedergibt;
M H oder ein kosmetisch verträgliches Gegenion mit einer Wertigkeit von 1 oder 2 wiedergibt;
a eine ganze Zahl von 1 bis 4 ist und
b 1 oder 2 ist, umfasst,
auf die Haut und/oder das Haar.

2. Kosmetisches Verfahren nach Anspruch 1, wobei das Acyllactylat dergestalt ist, dass M H oder ein Gegenion, ausgewählt aus der Gruppe, bestehend aus Natrium, Kalium oder Calcium, darstellt.

3. Kosmetisches Verfahren nach Anspruch 1 oder 2, wobei das Acyllactylat dergestalt ist, dass der Acylrest 8 oder 12 Kohlenstoffatome enthält.

4. Kosmetisches Verfahren nach Ansprüchen 1, 2 oder 3, wobei die Zusammensetzung weiterhin eine Substanz umfasst, die als Sonnenschutzmittel wirkt.

5. Kosmetisches Verfahren nach einem der Ansprüche 1-4, wobei die Zusammensetzung weiterhin einen Haarwuchsförderer umfasst.

6. Kosmetisches Verfahren nach einem der vorangehenden Ansprüche, wobei die Zusammensetzung weiterhin ein antientzündliches Mittel umfasst.

7. Kosmetisches Verfahren nach einem der vorangehenden Ansprüche, wobei der Haarausfall durch Propionibacterium acnes verursacht oder verschärft wird oder damit verbunden ist.

## Revendications

1. Procédé cosmétique d'empêchement et/ou de traitement de la chute des cheveux comprenant l'application à la peau et/ou aux cheveux d'une quantité efficace d'une composition cosmétique comprenant un acyl-lactylate en C6-12 ou son dérivé ayant la structure :
(RCO(O-CHCH₃-CO)ₐO)_{b} M
dans laquelle RCO représente un radical acyle en C6-12 saturé ; M représente H ou un contre-ion acceptable sur le plan cosmétique ayant une valence de 1 ou 2 ; a est un nombre entier de 1 à 4 (y compris) et b est 1 ou 2.

2. Procédé cosmétique selon la revendication 1, dans lequel l'acyl-lactylate est tel que M est H ou un contre-ion choisi parmi le sodium, le potassium ou le calcium.

3. Procédé cosmétique selon la revendication 1 ou 2, dans lequel l'acyl-lactylate est tel que le radical acyle contient 8 ou 12 atomes de carbone.

4. Procédé cosmétique selon la revendication 1, 2 ou 3, dans lequel la composition contient en outre une substance agissant comme un écran solaire.

5. Procédé cosmétique selon l'une quelconque des revendications 1 à 4, dans lequel la composition comprend de plus un promoteur de croissance capillaire.

6. Procédé cosmétique selon l'une quelconque des revendications précédentes, dans lequel la composition contient de plus un agent anti-inflammatoire.

7. Procédé cosmétique selon l'une quelconque des revendications précédentes, dans lequel la chute des cheveux est provoquée ou exacerbée par ou associé à Propionibacterium acnes.
